# NEW EUROPEAN PATENT SPECIFICATION

(11) **EP 0 566 118 B2**
(45) Date of publication and mention of the opposition decision: **17.10.2001**
(45) Mention of the grant of the patent: 17.09.1997
(21) Application number: 93106150.1
(22) Date of filing: 15.04.1993
(51) Int. Cl.: C08B 37/00, C08B 15/10, C08L 1/26, C08B 11/20

(54) **Process for the preparation of modified polysaccharides and modified polysaccharides**
Verfahren zur Herstellung von modifizierten Polysacchariden und modifizierte Polysaccharide
Procédé de préparation de polysaccharides modifiés; Polysaccharides modifiés

(30) Priority: 17.04.1992 US 870529
(43) Date of publication of application: 20.10.1993
(73) Proprietor: KIMBERLY-CLARK WORLDWIDE, INC., Neenah, Wisconsin 54956 (US)
(72) Inventor: Qin, Jian, Appleton, Wisconsin 54914 (US)
(74) Representative: Diehl, Hermann O. Th., Dr.

(56) References cited:
- EP-A- 0 538 904
- WO-A-90/10495
- CH-A- 491 140
- DD-A- 212 969
- GB-A- 1 086 323
- GB-A- 1 397 154
- GB-A- 2 027 714
- US-A- 469 700
- US-A- 3 589 364
- US-A- 4 090 013
- US-A- 4 697 008
- DATABASE WPI Week 8643, Derwent Publications Ltd., London, GB; AN 81-63016D & JP-A-56 084 701
- DATABASE WPI Week 9222, Derwent Publications Ltd., London, GB; AN 92-180940 & JP-A-4 120 142

## Description

The present invention relates to a method for producing modified polysaccharides and to specific modified polysaccharides.

The use of water-swellable, generally water-insoluble absorbent materials, commonly known as superabsorbents, in disposable absorbent personal care products is known. Such absorbent materials are generally employed in absorbent products such as diapers, training pants, adult incontinence products, feminine care products, and the like, in order to increase the absorbent capacity of such products while reducing their overall bulk Such absorbent materials are generally present in absorbent products in a fibrous matrix, such as a matrix of wood pulp fluff. A matrix of wood pulp fluff generally has an absorbent capacity of about 6 grams of liquid per gram of fluff. The absorbent materials described above generally have an absorbent capacity of at least about 10, preferably of about 20, and often of up to 100 times their weight in water. Clearly, incorporation of such absorbent materials in personal care products can reduce the overall bulk while increasing the absorbent capacity of such products.

A wide variety of materials have been described for use as absorbent materials in such personal care products. Such materials include natural-based materials such as agar, pectin, gums, carboxyalkyl starch, carboxyalkyl cellulose, and the like, as well as synthetic materials such as polyacrylates, polyacrylamides, hydrolyzed polyacrylonitrile, and the like. While the natural-based, absorbent materials are known for use in personal care products, they have not gained wide usage in such products. The natural-based, absorbent materials have not gained wide usage in personal care products, at least in part, because their absorbent properties are generally inferior compared to the synthetic absorbent materials, such as the polyacrylates. Specifically, many of the natural-based materials tend to form soft, gelatinous masses when swollen with a liquid. When employed in absorbent products, the presence of such soft gelatinous masses tends to prevent the transport of liquid within the fibrous matrix in which the absorbent materials are incorporated. This phenomenon is known as gel-blocking. Once gel-blocking occurs, subsequent insults of liquid cannot be efficiently absorbed by the product, and the product tends to leak. Further, many of the natural-based materials exhibit poor absorption properties, particularly when subjected to extemal pressures.

In contrast, the synthetic, absorbent materials are often capable of absorbing large quantities of liquid while maintaining a generally stiff, non-gelatinous character. Accordingly, the synthetic, absorbent materials can be incorporated in absorbent products while minimizing the likelihood of gel-blocking.

Carboxyalkyl cellulose materials and other modified polysaccharides are known in the art. As a general rule, carboxyalkyl cellulose materials are formed from a cellulosic material which has been treated with carboxyalkylating reactants, such as a chloroalkanoic acid, preferably monochloroacetic acid, and an alkali, such as sodium hydroxide, optionally, in the presence of an alcohol. Such a process is described, for example, in U.S. Patent 3,723,413, issued March 27, 1973, to Chatterjee et al. Such carboxyalkyl celluloses are generally water-soluble. Various methods of rendering such water-soluble carboxyalkyl celluloses water-insoluble are known.

U.S. Patent 2,639,239 issued May 19, 1953, to Elliott describes a process in which a commercially available water-soluble, alkali-metal salt of carboxymethyl cellulose having a degree of substitution of from about 0.5 to about 1 is subjected to a thermal treatment for up to 10 hours which renders such water-soluble carboxymethyl cellulose capable of forming highly swollen gel particles.

Similarly, U.S. Patent 3,723,413, discussed above, describes the heat-treatment of a carboxyalkyl cellulose in the presence of remaining carboxyalkylating reactants and by-products, such that the carboxyalkyl cellulose becomes water insoluble and possessed of desirable liquid absorptive and retentive properties and characteristics.

U.S. Patent 3,345,358 issued October 3, 1967, to Inklaar describes a method of preparing a gel-forming derivative of polysaccharides such as carboxymethyl starch. The method involves acidifying finely divided carboxymethyl ethers of polysaccharides by treating them with acid in methanol or other water-miscible organic liquid medium. In this manner, acid carboxymethyl groups are formed on the material. The material is held under acidified, non-hydrolyzing conditions to bring about the formation of ester bonds whereby constituent macromolecules of the material become crosslinked one to another. The material is then neutralized with an alkaline. The derivatives so produced are described as being capable of forming a gel upon addition to water.

U.S. Patent 3,379,720 issued April 23, 1968, to Reid describes a process of preparing modified polysaccharides such as ethers and esters of cellulose comprising slurrying a water-soluble polysaccharide in any inert medium, acidifying said polysaccharide, removing excess acid from the acidified polysaccharide, drying same and heat-curing.

U.S. Patent 4,689,408 issued August 25, 1987, to Gelman et al. describes a method of preparing salts of carboxymethyl cellulose. The method involves treating a carboxymethyl cellulose with water, adding a nonsolvent for the carboxymethyl cellulose, and recovering the carboxymethyl cellulose. The carboxymethyl cellulose is said to have an absorbency of at least 25 grams of liquid per gram of carboxymethyl cellulose.

JP-A-56 084701 describes the reaction of (I) a cationic polysaccharide (e.g. glycol chitosane or aminocellulose) with (II) an anionic polysaccharide (e.g. CMC) by (1) mixing an aqueous solution of (I) with an aqueous solution of (II), (2) adding a solvent selected from (m)ethanol, isopropanol or acetone and (3) separating and drying the precipitate. There is no teaching in that prior art that the reaction should be carried out at a higher temperature.

Unfortunately, the known modified polysaccharide materials do not possess absorptive properties comparable to many of the synthetic, highly absorptive materials. This has prevented widespread use of such carboxyalkyl polysaccharides in absorbent personal care products.

It is therefore desirable to develop and produce a natural-based, highly absorbent material having absorptive properties similar to the synthetic, highly absorptive materials and, thus, suitable for use in personal care absorbent products.

The present invention provides a method for producing a water-swellable, water-insoluble modified polysaccharide according to independent claim 1 or 13. Further advantageous features, aspects and details of the method are evident from the dependent claims, the description, examples and drawings. The method according to a specific aspect comprises the steps of forming a mixture comprising a water-soluble modified polysaccharide, water, and a crosslinking agent. The modified polysaccharide is recovered from said solution and heat-treated at a temperature between 100 and 200°C for 20 to 100 min. to crosslink the modified polysaccharide.

The present invention further provides a water-swellable, water-insoluble modified polysaccharide according to independent claim 14 or 16. Further advantageous features, aspects, and details of this modified polysaccharide are evident from the dependent claims, the description, examples and the drawings. The modified polysaccharide according to a specific aspect is characterized in that it possesses both ester and amide crosslinks.

The present invention provides modified polysaccharides having improved absorbent properties. Specifically, the present invention provides modified polysaccharides having an improved ability to absorb liquid while under a load and a process for the preparation thereof.

Fig. 1 illustrates the apparatus for determining the Absorbency Under Load values of an absorbent material.

Fig. 2 illustrates, in the form of a graph, the results of the physical property testing set forth in Table 2.

Fig. 3 illustrates, in the form of a graph, the results of the physical property testing set forth in Table 3.

In one aspect, the present invention concerns a method for producing a water-swellable, water-insoluble modified polysaccharide. The method comprises the steps of forming a mixture comprising a modified polysaccharide, water, and a crosslinking agent. The modified polysaccharide is recovered from the mixture and heat-treated at a temperature between 100 and 200°C for 20 to 100 min. to crosslink the modified polysaccharide.

Modified polysaccharides suitable for use in the present invention are generally water soluble. As used herein, a modified polysaccharide will be considered to be water-soluble when it dissolves in water to form a true solution.

As a general rule, the water-soluble modified polysaccharides will be free from a substantial degree of crosslinking, as crosslinking tends to render the modified polysaccharides water insoluble.

Modified polysaccharides suitable for use in the present invention include the carboxylated, sulfonated, sulfated, and phosphated derivatives of polysaccharides, their salts, and mixtures thereof. Exemplary of suitable polysaccharides are cellulose, starch, carrageenan, agar, gellan gum, and chitin, as well as mixtures thereof. The preferred modified polysaccharide is a carboxyalkyl polysaccharide.

Suitable carboxyalkyl polysaccharides for use in the present invention include carboxyalkyl celluloses such as carboxymethyl cellulose, carboxyethyl cellulose, carboxyalkyl carrageenan, carboxyalkyl agar, and carboxyalkyl·gellan gum, as well as mixtures thereof. The preferred carboxyalkyl polysaccharide is a carboxyalkyl cellulose with the preferred carboxyalkyl cellulose being carboxymethyl cellulose.

Methods of making carboxyalkyl polysaccharides are known to those skilled in the art. Suitably, a polysaccharide material such as wood pulp fluff, cotton, cotton linters, starch, and agar are provided. The polysaccharide material may be in the form of fibers or of fibers which have been comminuted to particulate form. The polysaccharide material is dispersed in an inert solvent such as an alcohol and carboxyalkylating reagents added to the dispersion. Carboxyalkylating reagents generally comprise a chloroalkanoic acid, such as monochloroacetic acid, and sodium hydroxide.

It is to be understood that it may be possible to perform the modification of the starting polysaccharide in such a manner that a solution of carboxyalkyl polysaccharide and water can be formed directly, without the need of an intermediate recovery step. For example, the modification process may be performed under low moisture conditions. That is, the starting polysaccharide may be wetted with, for example, 1 part of water for each part of starting polysaccharide. Carboxyalkylating reagents can then be mixed with the wetted starting polysaccharide such that carboxyalkylation occurs. Additional water can then be added to the modified polysaccharide to form a solution of modified polysaccharide and water. In this manner, no recovery step is necessary between formation of the modified polysaccharide and the formation of the solution of modified polysaccharide, water and crosslinking agent. If too much water is present in the starting polysaccharide, the carboxyalkylation reaction may not occur to a sufficient degree.

When the modified polysaccharide is a carboxyalkyl cellulose, the carboxyalkyl celluloses suitable for use in the present invention generally have an average degree of substitution from about 0.3 to about 1.5, preferably from about 0.4 to about 1.2. The degree of substitution refers to the average number of carboxyl groups present on the anhydroglucose unit of the cellulosic material. When the carboxyalkyl celluloses have an average degree of substitution within the range of from about 0.3 to about 1.5, the carboxyalkyl celluloses are generally water soluble.

Carboxyalkyl cellulose is available in a wide range of molecular weights. Carboxyalkyl cellulose having a relatively high molecular weight is often preferred for use in the present invention. Nonetheless, a wide range of molecular weights are suitable for use in the present invention. It is generally most convenient to express the molecular weight of a carboxyalkyl cellulose in terms of its viscosity in a 1.0 weight percent aqueous solution at 25°C Carboxymethyl celluloses suitable for use in the present invention will generally have a viscosity in a 1.0 weight percent aqueous solution of from about 10 (mPa) centipoise to about 80,000 mPa (centipoise), preferably from about 500 mPa (centipoise) to about 80,000 mPa (centipoise), and most preferably from about 1,000 mPa (centipoise) to about 80,000 mPa (centipoise).

Suitable carboxyalkyl celluloses are commercially available from numerous vendors. Exemplary of a commercially available carboxyalkyl cellulose is carboxymethyl cellulose, commercially available from Aqualon Company under the trade designation Aqualon™ or Blanose™ Cellulose Gum.

Crosslinking agents suitable for use in the present invention are generally water soluble. The crosslinking agent is suitably an organic compound having at least two functional groups capable of reacting with the carboxyl or hydroxyl group of a polysaccharide. It is desired that the crosslinking agent be selected from the group consisting of diamines, polyamines, diols, and polyols and mixtures thereof: particularly from the group consisting of primary diols, primary polyols, primary diamines and primary polyamines and mixtures thereof. Of the diols and polyols, those possessing longer carbon chain lengths (≥4) are generally preferred. Specifically, the crosslinking agent may be selected from the group consisting of chitosan glutamate, type A gelatin, diethylenetriamine, ethylene glycol, butylene glycol, polyvinyl alcohol, hyaluronic acid, polyethylene imine and their derivatives and mixtures thereof.

The mixture of modified polysaccharide, water, and crosslinking agent suitably comprises from about 0.01 to about 90 weight percent, beneficially from about 0.01 to about 30 weight percent, and preferably from about 2 to about 25 weight percent, based on total mixture weight, of the modified polysaccharide. The mixture of modified polysaccharide, water and crosslinking agent suitably comprises from about 99.98 to about 10 weight percent, beneficially from about 99.98 to about 70 weight percent, and preferably from about 98 to about 75 weight percent water.

The modified polysaccharide is suitably dissolved in a solvent comprising at least about 30 weight percent water, beneficially about 50 weight percent water, preferably about 75 weight percent water, and most preferably 100 weight percent water. When a co-solvent is employed with the water, other suitable solvents include methanol, ethanol, and acetone.

The crosslinking agent is added to the mixture of modified polysaccharide and water in an amount of from about 0.01 to about 20, beneficially of from about 0.05 to about 10, and preferably of from about 0.1 to about 5 weight percent, based on total weight of the modified polysaccharide present in the mixture.

The mixture of modified polysaccharide, water, and crosslinking agent can be formed at any temperature at which the modified polysaccharide is soluble in the water. Generally, such temperatures will be within the range of from about 10°C to about 100°C. As a general rule, it is preferred to form the mixture of modified polysaccharide, water, and crosslinking agent with agitation.

The mixture of modified polysaccharide, water, and crosslinking agent, must be basic (pH >7). to provide the mixture of modified polysaccharide, water, and crosslinking agent with a basic pH, a base such as an aqueous solution of sodium hydroxide, potassium hydroxide, ammonia, or the like can be added to the mixture.

The mixture of modified polysaccharide, water, and crosslinking agent will suitably have a basic pH to about 12, beneficially to about 9, and preferably to about 8. The recovered modified polysaccharide will generally have the same pH as the mixture. However, Applicant has noted that the evaporative drying step tends to lower the pH of the recovered modified polysaccharide.

When the modified polysaccharide of the present invention is intended for use in personal care products, such as diapers, training pants, feminine care products, and the like, it is generally desired that the modified polysaccharide have a generally neutral character. For this reason, the recovered modified polysaccharide that is basic may be neutralized. The recovered modified polysaccharide which is basic may be neutralized, for example, by contacting with an acidic gas such as carbon dioxide.

After forming the mixture of modified polysaccharide, water, and crosslinking agent, the modified polysaccharide is recovered from the mixture. The evaporative drying recovers the modified polysaccharide from the mixture, without unacceptably deteriorating the absorption characteristics of the modified polysaccharide.

As a general rule, the modified polysaccharide can be recovered by evaporative drying at a temperature within the range of from about 10°C to about 100°C, preferably from about 50°C to about 80°C. Naturally, higher temperatures can be employed if the mixture is placed under pressure. Lower temperatures can be employed if the mixture is placed under a vacuum.

Depending on the form in which the modified polysaccharide is recovered, it may be necessary or desirable to alter the form of the modified polysaccharide. The modified polysaccharide may be recovered in the form of a film or sheet. It may be desirable to comminute the film or sheet material into particles or flakes of material.

The form of the recovered modified polysaccharide desired will depend to a large extent on the use for which it is intended. When the modified polysaccharide is intended for use in absorbent personal care products, it is generally desired that the modified polysaccharide be in the form of a discrete particle, fiber or flake. When in the form of a particle, it is generally desired that the particle have a maximum cross-sectional dimension within the range from about 50 micrometers to about 2,000 micrometers, preferably within the range from about 100 micrometers to about 1,000 micrometers, most preferably within the range from about 300 micrometers to about 600 micrometers.

The recovered modified polysaccharide is then heat-treated at an elevated temperature to crosslink the modified polysaccharide. The modified polysaccharide will be heat-treated at a temperature within the range from 100°C to 200°C, and preferably from 100°C to 160°C. The higher the temperature employed, the shorter the period of time generally necessary to achieve the desired degree of crosslinking. Generally, the heat-treating process will extend over a time period within the range of from 20 minutes to 100 minutes.

If you provide the mixture of modified polysaccharide, water, and crosslinking agent contrary to the present invention with an acidic character, the time necessary to effect the crosslinking can be shortened. Providing the mixture of modified polysaccharide, water, and crosslinking agent with a slightly basic character tends to lengthen the time of the crosslinking process, at a given temperature, compared to a slightly acidic or neutral mixture.

The heat-treating process causes the modified polysaccharide to cross link and become generally water insoluble. Without intending to be bound thereby, Applicant hypothesizes that the heat-treating process causes the modified polysaccharide to undergo a degree of self-crosslinking, not related to the presence of the crosslinking agent, through the formation of ester linkages. This self-crosslinking is in addition to the crosslinking caused by the presence of the crosslinking agent. Further, when the crosslinking agent is a diamine or polyamine, Applicant hypothesizes that crosslinking occurs through amidation of the carboxyl group through the formation of an ammonia salt. Esterification, through a self-crosslinking process, is believed to occur primarily under weakly acidic, neutral, or slightly basic condition, wherein the weakly acidic or neutral conditions are not within the range of the present invention. Esterification, through a self-crosslinking process, is not believed to proceed to a significant degree under relatively basic conditions. Crosslinking due to the crosslinking agent may occur under both acidic and basic conditions. Thus, the presence of the crosslinking agent allows for crosslinking to occur over a broad pH range.

The heat-treating process desirably produces a modified polysaccharide having the ability to absorb a liquid while the modified polysaccharide is under a load. Synthetic polymeric materials, such as polyacrylates, having a generally high ability to absorb while under a load, have been found to minimize the occurrence of gel-blocking when incorporated in absorbent products. The method by which the Absorbency Under Load is determined is set forth below in connection with the examples. The Absorbency Under Load values determined as set forth below and reported herein refer to the amount, in grams, of an aqueous solution containing 0.9 weight percent sodium chloride a gram of the modified polysaccharide can absorb in 60 minutes under a load of about about 0.02 kg/cm² (about 0.3 pounds per square inch). When the modified polysaccharide is a carboxyalkyl cellulose, the carboxyalkyl cellulose has an Absorbency Under Load (AUL) of at least 17, beneficially of at least 20, most beneficially of at least 24, and preferably of at least 27 grams per gram.

Applicant has found that there is generally an optimum combination of time and temperature at which to crosslink and optimize the Absorbency Under Load of a particular modified polysaccharide. If too little crosslinking occurs, the modified polysaccharide may possess a relatively low Absorbency Under Load due to a lack of gel strength. If too much crosslinking occurs, the modified polysaccharide may similarly have a relatively low Absorbency Under Load due to the inability of the carboxyalkyl cellulose to absorb liquid.

The presence of the crosslinking agent, particularly a diamine or polyamine, may improve the Absorbency Under Load of a modified polysaccharide according to the present invention when compared to an identical modified polysaccharide without a crosslinking agent. Specifically, the modified polysaccharides according to the present invention may suitably have an Absorbency Under Load of at least 10 percent, and preferably at least 20 percent greater than an identical modified polysaccharide without a crosslinking agent. The identical modified polysaccharide will have the same composition and be formed and heat-treated exactly like the modified polysaccharide of the present invention, except it will not comprise the crosslinking agent.

The presence of the crosslinking agent may improve the aging characteristics of the modified polysaccharides with respect to Absorbency Under Load. Modified polysaccharides produced with crosslinking agents according to the method of the present invention will tend to retain their AUL after aging. Specifically, modified polysaccharides produced with crosslinking agents according to the present invention may retain greater than 50 percent, and preferably greater than 70 percent, of their AUL value after aging for 60 days at 24°C and 30% relative humidity. For example, if a modified polysaccharide produced with crosslinking agents according to the present invention has an AUL of 20 immediately after heat-treatment, that modified polysaccharide may have an AUL of at least 10, and preferably of 14, after aging for 60 days at 24°C and 30% relative humidity. Identical modified polysaccharides not comprising the crosslinking agent tend not to retain their AUL after aging.

In another aspect, the present invention relates to a water-swellable, generally water-insoluble modified polysaccharide characterized in that said modified polysaccharide possesses crosslinks formed by esterification and amidation. Such a modified polysaccharide is formed by the method described above.

As discussed above, the crosslinks formed by esterification may result from self-crosslinking of the material and/or from the presence of a crosslinking agent other than a diamine or polyamine. The crosslinks formed by amidation result from the presence of a diamine or polyamine crosslinking agent.

As described above, the modified polysaccharide is suitably a carboxyalkyl polysaccharide, such as a carboxyalkyl cellulose (e.g., carboxymethyl cellulose and carboxyethyl cellulose). Such a carboxyalkyl cellulose has an Absorbency Under Load value of at least 17, beneficially of at least 20, preferably of at least 24, and most preferably of at least 27.

Those skilled in the art will recognize that the presence of crosslinks formed by esterification and amidation can be detected through various analytical techniques. For example, infrared spectroscopy, nuclear magnetic resonance, and the like can be used to verify the presence of ester and amide crosslinks.

The modified polysaccharides of the present invention are suitable for use in personal care products such as diapers, training pants, feminine care products, adult incontinent products, wound dressings, and the like.

### Test Methods

### Absorbency Under Load

The Absorbency Under Load (AUL) is a test which measures the ability of an absorbent material to absorb a liquid (0.9 weight percent solution of sodium chloride in distilled water) while under an applied load or restraining force.

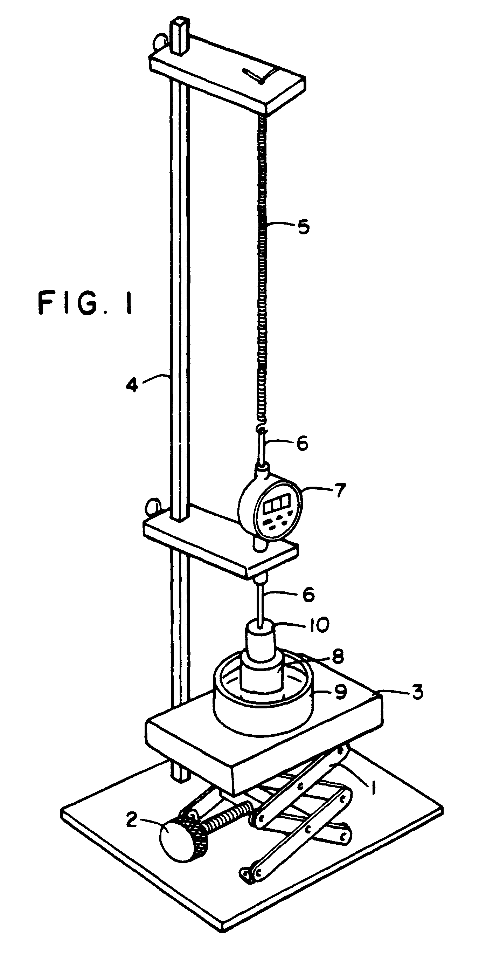

Referring to Fig. 1, the apparatus and method for determining AUL will be described. Shown is a perspective view of the apparatus in position during a test. Shown is a laboratory jack 1 having an adjustable knob 2 for raising and lowering the platform 3. A laboratory stand 4 supports a spring 5 connected to a modified thickness meter probe 6, which passes through the housing 7 of the meter, which is rigidly supported by the laboratory stand. A plastic sample cup 8, which contains the superabsorbent material sample to be tested, has a liquid-permeable bottom and rests within a Petri dish 9, which contains the saline solution to be absorbed. A weight 10 rests on top of a spacer disc (not visible) resting on top of the superabsorbent material sample (not visible).

The sample cup consists of a plastic cylinder having a 2.54 cm (1 inch) inside diameter and an outside diameter of 3.175 cm (1.25 inch). The bottom of the sample cup is formed by adhering a 0.149 mm (100 mesh) metal screen having 150 µm openings to the end of the cylinder by heating the screen above the melting point of the plastic and pressing the plastic cylinder against the hot screen to melt the plastic and bond the screen to the plastic cylinder.

The modified thickness meter used to measure the expansion of the sample while absorbing the saline solution is a Mitutoyo Digimatic Indicator, IDC Series 543, Model 543-180, having a range of 0.0 - 1.27 cm (0-0.5 inch) and an accuracy of 0.000127 cm (0.00005 inch) (Mitutoyo Corporation, 31-19, Shiba 5-chome, Minato-ku, Tokyo 108, Japan). As supplied from Mitutoyo Corporation, the thickness meter contains a spring attached to the probe within the meter housing. This spring is removed to provide a free-falling probe which has a downward force of about 27 grams. In addition, the cap over the top of the probe, located on the top of the meter housing, is also removed to enable attachment of the probe to the suspension spring 5 (available from McMaster-Carr Supply Co., Chicago, Illinois, Item No. 9640K41), which serves to counter or reduce the downward force of the probe to about 1 gram, ± 0.5 gram. A wire hook can be glued to the top of the probe for attachment to the suspension spring. The bottom tip of the probe is also provided with an extension needle (Mitutoyo Corporation, Part No. 131279) to enable the probe to be inserted into the sample cup.

To carry out the test, a 0.160 gram sample of the absorbent material, which has been sieved to a particle size between 300 and 600 µm, is placed into the sample cup. The sample is then covered with a plastic spacer disc, weighing 4.4 grams, which is slightly smaller than the inside diameter of the sample cup and serves to protect the sample from being disturbed during the test. The 100 gram weight is then placed on top of the spacer disc, thereby applying a load of 0.02 kg/cm² (0.3 pounds per square inch). The sample cup is placed in the Petri dish on the platform of the laboratory jack raised up until it contacts the tip of the probe. The meter is zeroed. A sufficient amount of saline solution is added to the Petri dish (50-100 milliliters) to begin the test. The distance the weight is raised by the expanding sample as it absorbs the saline solution is measured by the probe. This distance, multiplied by the cross-sectional area inside the sample cup, is a measure of the expansion volume of the sample due to absorption. Factoring in the density of the saline solution and the weight of the sample, the amount of saline solution absorbed is readily calculated. The weight of saline solution absorbed after 60 minutes is the AUL value, expressed as grams saline solution absorbed per gram of absorbent. If desired, the readings of the modified thickness meter can be continuously input to a computer (Mitutoyo Digimatic Miniprocessor DP-2 DX) to make the calculations and provide AUL readings, As a cross-check, the AUL can also be determined by determining the weight difference between the sample cup before and after the test, the weight difference being the amount of solution absorbed by the sample.

Of the following examples only those wherein the mixture comprising the water - soluble modified polysaccharide, water, and crosslinking agent is basic (ph > 7) are embodiments of the invention.

### Examples

### Example 1

Two sodium carboxymethyl celluloses (CMC) commercially available from the Aqualon Company under the trade designation AQUALON® Cellulose Gum CMC-7HCF or CMC-9H4F are provided. The CMC-7HCF has an average degree of substitution of about 0.7 and a viscosity in a 1 percent aqueous solution at 25°C. of about 1000-2800 mPa (centipoise). The CMC-9H4F has an average degree of substitution of about 0.9 and a viscosity in a 1 percent aqueous solution at 25°C. of about 2500-6000 mPa (centipoise). Each carboxymethyl cellulose is individually dissolved in distilled water to form a solution containing 2 weight percent carboxymethyl cellulose based on total solution weight. A crosslinking agent is dissolved in water to form a solution containing 0.5 weight percent of the crosslinking agent. The crosslinking agents employed are chitosan glutamate, commercially available from Protan Biopolymer A/S, Norway, under the trade designation Sea Cure G: 1, 4-butylene glycol, commercially available from the Aldrich Chemical Company; polyethylene imine (molecular weight 50,000 - 100,000) commercially available from Polysciences, Inc.; sodium salt of hyaluronic acid commercially available from Sigma; Type A gelatin commercially available from the Aldrich Chemical Company under the trade designation 300 Bloom; and diethylene triamine commercially available from the Aldrich Chemical Company. The aqueous solution containing the crosslinking agent is then added to the individual aqueous solutions containing the carboxymethyl cellulose to provide various concentrations of crosslinking agent based on total weight of the carboxymethyl cellulose present in the aqueous solution. The resulting mixtures containing water, carboxymethyl cellulose, and crosslinking agent are then thoroughly mixed. The carboxymethyl cellulose is recovered from the solution by evaporative drying at 80°C. in a Blue M air-convection oven. After drying, the recovered carboxymethyl cellulose is ground into granules in a blender and heat-treated at various times and temperatures in an oven. Various combinations of carboxymethyl cellulose, crosslinking agent, concentration of crosslinking agent, heat-treatment temperature and heat-treatment time are made. The Absorbency Under Load values of the various carboxymethyl celluloses so prepared are measured. The exact combination of carboxymethyl cellulose and crosslinking agent and its AUL value are set forth in Table 1. Similarly, control samples of the carboxymethyl cellulose CMC-7HCF and CMC-9H4F are tested for AUL values. These results are also set forth in Table 1.

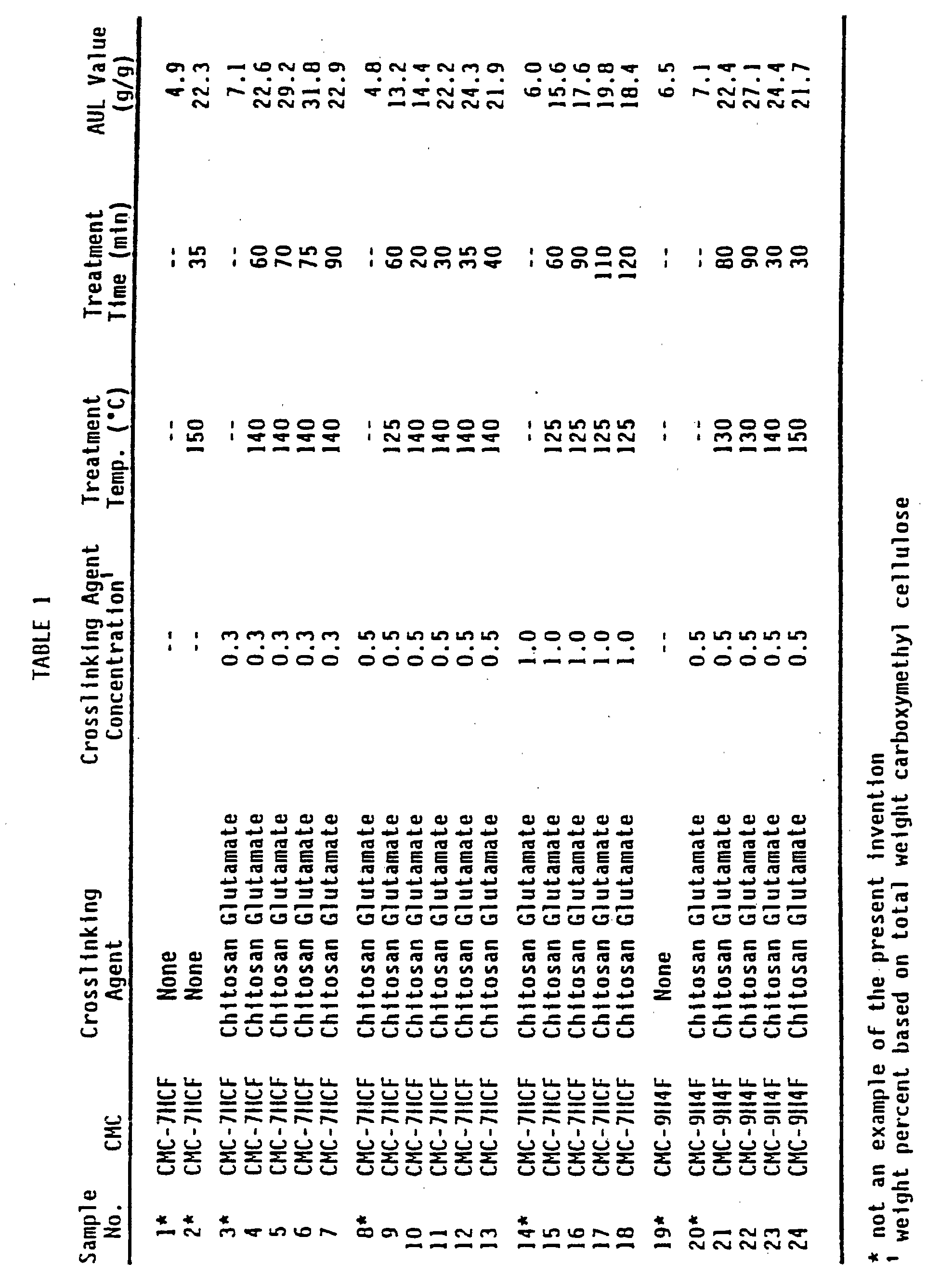

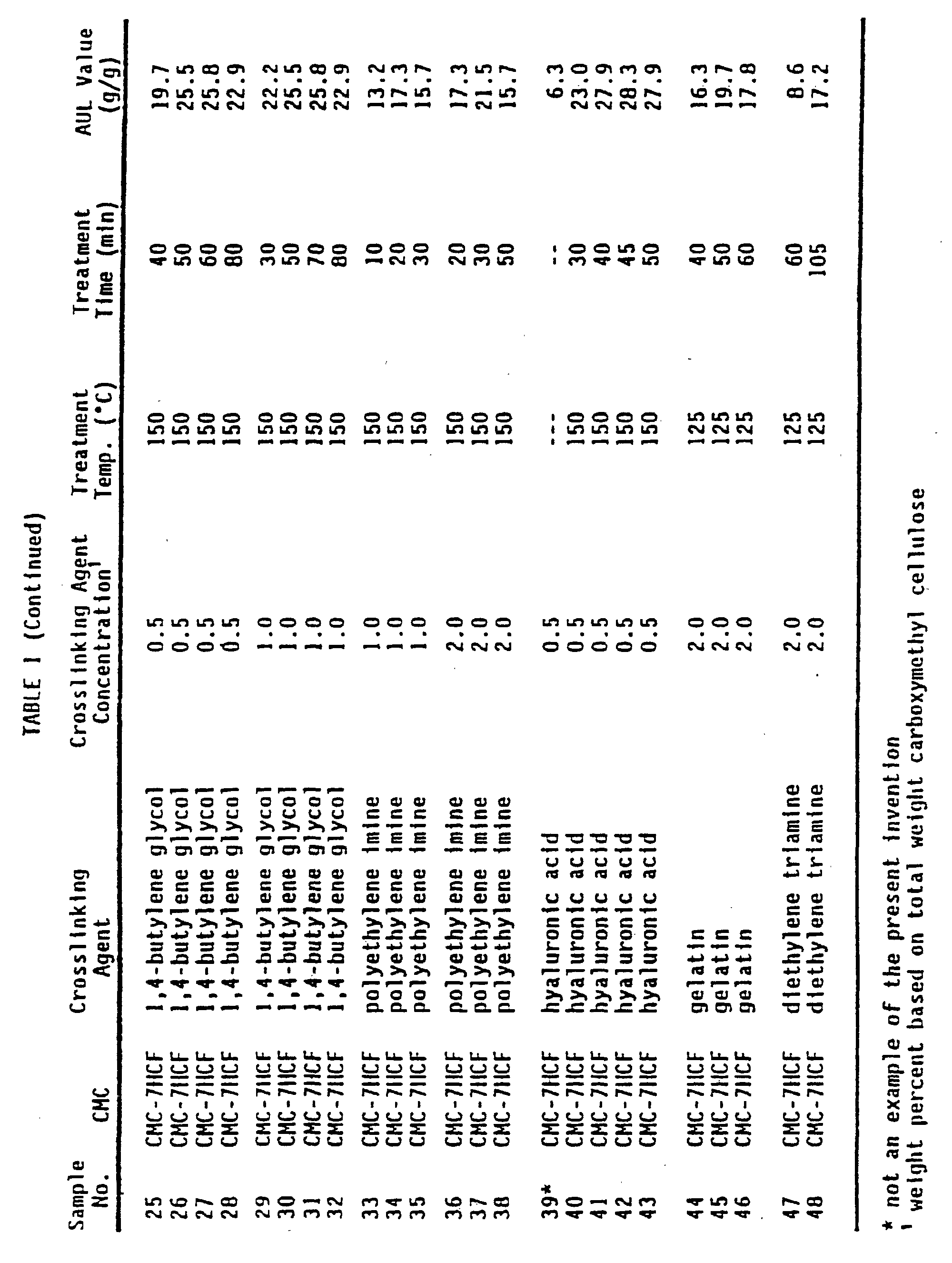

As can be seen from reference to Table 1, the method according to the present invention significantly increases the Absorbency Under Loac value of the starting carboxymethyl cellulose materials. All of the crosslinking agents employed are effective to increase the Absorbency Under Load values. Further, it is seen that the crosslinking agents are effective over a range of concentrations.

### Example 2

The chitosan glutamate aqueous solution employed in Example 1 is weakly acidic. In order to evaluate the effect of pH, a basic crosslinker (diethylenetriamine, commercially available from the Aldrich Chemical Company), is used. Again, a carboxymethyl cellulose (CMC-7HCF) is dissolved in distilled water to form a 2 weight percent aqueous solution. The diethylenetriamine is dissolved in water to form a 0.5 weight percent aqueous solution. The aqueous solution of diethylenetriamine is then added to the aqueous solution of carboxymethyl cellulose to provide a diethylenetriamine concentration of 2.0 weight percent based on total weight of the carboxymethyl cellulose present in the aqueous solution. The carboxymethyl cellulose is then recovered by evaporative drying and comminuted into particles as described in Example 1. A comparison material is prepared by dissolving carboxymethyl cellulose (CMC-7HCF) in distilled water to form a 2 weight percent solution. To the aqueous solution of carboxymethyl cellulose is then added 0.004 weight percent sodium hydroxide. The comparison carboxymethyl cellulose is recovered and comminuted into particles as described in Example 1. Both samples are then heated at various temperatures for 30 minutes. The resulting polymers are tested for Absorbency Under Load. The results of the testing are set forth in Table 2.

**TABLE 2**

| Sample No. | Composition | Treatment Temp (°C) | Treatment Time (min.) | AUL Value (g/g) |
|---|---|---|---|---|
| 49 | CMC/diethylenetriamine | 80 | 30 | 6.2 |
| 50 | CMC/diethylenetriamine | 110 | 30 | 6.2 |
| 51 | CMC/diethylenetriamine | 120 | 30 | 6.5 |
| 52 | CMC/diethylenetriamine | 130 | 30 | 7.7 |
| 53 | CMC/diethylenetriamine | 140 | 30 | 13.1 |
| 54 | CMC/diethylenetriamine | 150 | 30 | 17.9 |
| 55 | CMC/diethylenetriamine | 160 | 30 | 15.9 |
| 56 | CMC/diethylenetriamine | 170 | 30 | 14.0 |
| 57* | CMC/sodium hydroxide | 80 | 30 | 6.1 |
| 58* | CMC/sodium hydroxide | 110 | 30 | 6.0 |
| 59* | CMC/sodium hydroxide | 120 | 30 | 5.6 |
| 60* | CMC/sodium hydroxide | 130 | 30 | 6.0 |
| 61* | CMC/sodium hydroxide | 140 | 30 | 5.9 |
| 62* | CMC/sodium hydroxide | 150 | 30 | 5.3 |
| 63* | CMC/sodium hydroxide | 160 | 30 | 5.4 |
| 64* | CMC/sodium hydroxide | 180 | 30 | 4.3 |

| | | | | |
|---|---|---|---|---|
| *Not an example of the present invention. | | | | |

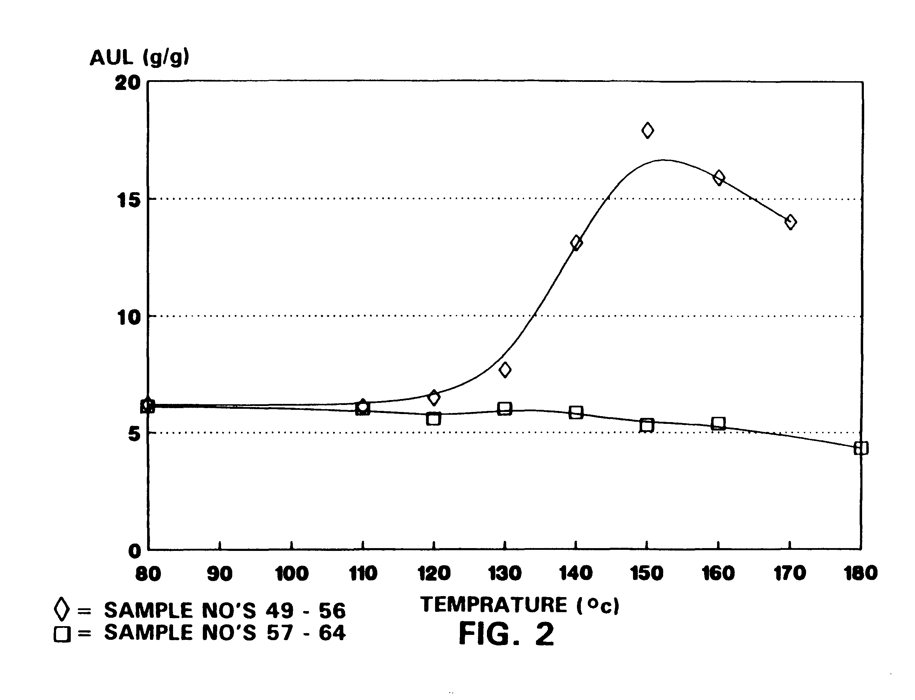

The results set forth in Table 2 are graphically illustrated in Fig. 2. As can be seen from reference to Fig. 2 and Table 2, the carboxymethyl cellulose, under basic conditions, exhibits no improvement in Absorbency Under Load values through the heat-treating step. In contrast, the carboxymethyl cellulose containing the basic crosslinker, diethylene triamine, is seen to exhibit an improvement in AUL value as a result of the heat-treating step. This indicates that a self-crosslinking of the carboxymethyl cellulose does not readily occur at relatively basic pH.

### Example 3

Sample Nos. 65-71 are prepared by forming an aqueous solution containing 2 weight percent of carboxymethyl cellulose (CMC-7HCF). To the aqueous solution is added an amount of sodium hydroxide sufficient to bring the pH of the solution to 9. No crosslinking agent is present in the solution. The carboxymethyl cellulose is then, according to the method of Example 1, recovered, comminuted, heat-treated at 150°C. for various times, and tested for Absorbency Under Load.

Sample Nos. 72-77 are prepared by forming an aqueous solution comprising 2 weight percent carboxymethyl cellulose (CMC-7HCF). To the solution is then added 0.5 weight percent chitosan glutamate in the manner set forth in Example 1. The solution is found to have a pH of about 7.4. The carboxymethyl cellulose is then, according to the method of Example 1, recovered, comminuted, heat-treated at 150°C. for various times and tested for Absorbency Under Load.

Sample Nos. 78-103 are prepared in the same manner as Sample Nos. 72-77, except sodium hydroxide is added to the aqueous solution containing carboxymethyl cellulose and chitosan glutamate prior to recovery. The amount of sodium hydroxide added is sufficient to bring the pH of the solution to 9.2, 10.1, or 10.9. The carboxymethyl cellulose is then, according to the method of Example 1, recovered, comminuted, heat-treated at 150°C. for various times and tested for Absorbency Under Load.

The results of this testing (sample nos. 65-103) are set forth in Table 3.

**TABLE 3**

| Sample No. | Crosslinking Agent | pH | Treatment Temp (°C) | Treatment Time (min) | AUL Value (g/g) |
|---|---|---|---|---|---|
| 65* | None | 9.0 | -- | -- | 4.9 |
| 66* | None | 9.0 | 150 | 30 | 6.9 |
| 67* | None | 9.0 | 150 | 50 | 6.9 |
| 68* | None | 9.0 | 150 | 80 | 6.6 |
| 69* | None | 9.0 | 150 | 110 | 6.5 |
| 70* | None | 9.0 | 150 | 150 | 6.6 |
| 71* | None | 9.0 | 150 | 180 | 6.4 |
| 72* | Chitosan glutamate | 7.4 | - | -- | 4.8 |
| 73 | Chitosan glutamate | 7.4 | 150 | 15 | 11.1 |
| 74 | Chitosan glutamate | 7.4 | 150 | 22 | 19.6 |
| 75 | Chitosan glutamate | 7.4 | 150 | 30 | 22.4 |
| 76 | Chitosan glutamate | 7.4 | 150 | 45 | 20.2 |
| 77 | Chitosan glutamate | 7.4 | 150 | 60 | 17.8 |
| 78* | Chitosan glutamate | 9.2 | -- | -- | 4.9 |
| 79 | Chitosan glutamate | 9.2 | 150 | 15 | 7.3 |
| 80 | Chitosan glutamate | 9.2 | 150 | 30 | 10.6 |
| 81 | Chitosan glutamate | 9.2 | 150 | 45 | 19.5 |
| 82 | Chitosan glutamate | 9.2 | 150 | 60 | 23.5 |
| 83 | Chitosan glutamate | 9.2 | 150 | 70 | 24.1 |
| 84 | Chitosan glutamate | 9.2 | 150 | 80 | 23.2 |
| 85 | Chitosan glutamate | 9.2 | 150 | 120 | 25.0 |
| 86 | Chitosan glutamate | 9.2 | 150 | 150 | 26.5 |
| 87 | Chitosan glutamate | 9.2 | 150 | 180 | 27.4 |
| 88 | Chitosan glutamate | 9.2 | 150 | 240 | 21.6 |
| 89* | Chitosan glutamate | 10.1 | -- | -- | 4.9 |
| 90 | Chitosan glutamate | 10.1 | 150 | 30 | 7,1 |
| 91 | Chitosan glutamate | 10.1 | 150 | 60 | 8.3 |
| 92 | Chitosan glutamate | 10.1 | 150 | 120 | 16.1 |
| 93 | Chitosan glutamate | 10.1 | 150 | 150 | 19.3 |
| 94 | Chitosan glutamate | 10.1 | 150 | 180 | 21.3 |
| 95 | Chitosan glutamate | 10.1 | 150 | 210 | 20.5 |
| 96 | Chitosan glutamate | 10.1 | 150 | 240 | 23.0 |
| 97* | Chitosan glutamate | 10.9 | -- | -- | 4.9 |
| 98 | Chitosan glutamate | 10.9 | 150 | 60 | 7.2 |
| 99 | Chitosan glutamate | 10.9 | 150 | 110 | 9.6 |
| 100 | Chitosan glutamate | 10.9 | 150 | 120 | 12.3 |
| 101 | Chitosan glutamate | 10.9 | 150 | 150 | 13.8 |
| 102 | Chitosan glutamate | 10.9 | 150 | 180 | 15.8 |
| 103 | Chitosan glutamate | 10.9 | 150 | 240 | 19.4 |

| | | | | | |
|---|---|---|---|---|---|
| *Not an example of the present invention. | | | | | |

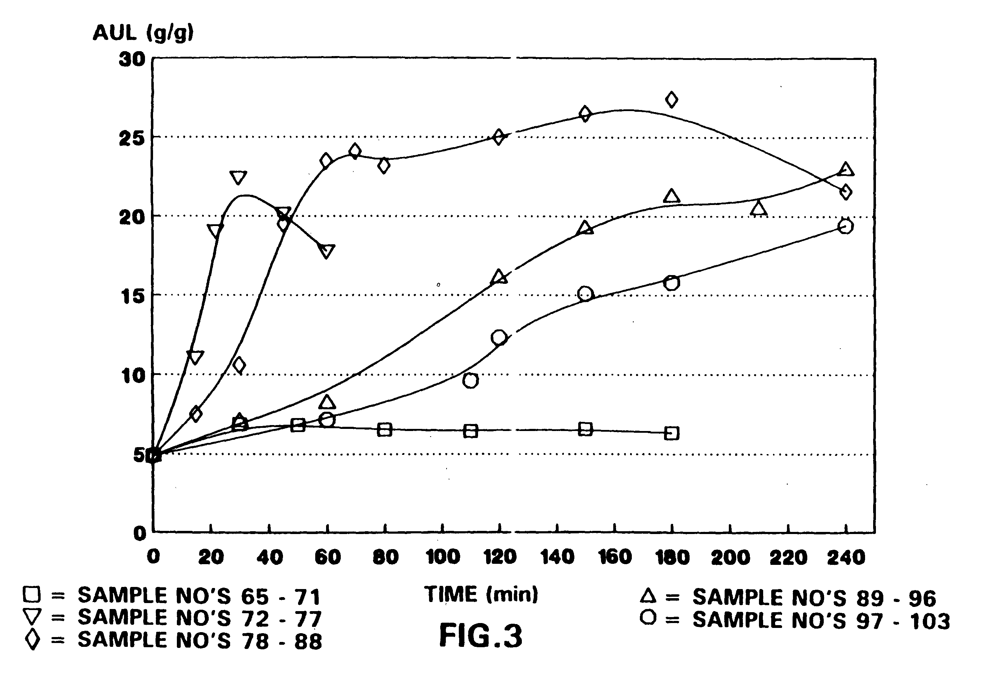

Fig. 3 graphically illustrates the results set forth in Table 3.

As can be seen from reference to Fig. 3 and Table 3, basic carboxymethyl cellulose containing no crosslinking agent (Sample Nos. 65-71) exhibits no significant improvement in Absorbency Under Load values as a result of heat-treatment. In contrast, Sample Nos. 72-103 show improvements in AUL values. It is seen that at a lower pH, the heat-treatment time required to optimize AUL values is shorter than at a higher pH.

### Example No. 4

A carboxymethyl cellulose commercially available from Aqualon Company under the trade designation AQUALON® Cellulose Gum CMC-7L is provided. This carboxymethyl cellulose has a relatively low molecular weight exhibiting a viscosity in a 2 percent aqueous solution at 25°C. of about 25-50 mPa (centipoise). Sample No. 104 (comparative) is prepared by forming an aqueous solution containing 2 weight percent of the carboxymethyl cellulose (CMC-7L). The carboxymethyl cellulose is recovered and dried as described in Example 1. The material is found to have an Absorbency Under Load value of 2.1. Sample No. 105 (comparative) is prepared in the same manner as Sample No. 104 with the exception that, after recovery, the material is comminuted and heat-treated at 170°C. for 160 minutes. The resultant material is found to have an Absorbency Under Load value of 8.6. Neither material contains a crosslinking agent.

Sample No. 106 is prepared by forming an aqueous solution containing 2 weight percent of the carboxymethyl cellulose (CMC-7L). To the aqueous solution is added 1 weight percent chitosan glutamate in the manner set forth in Example 1. The carboxymethyl cellulose is then recovered and comminuted as set forth in Example 1. The resultant material is then heat-treated at 170° for two hours. The resulting material is found to have an Absorbency Under Load value of about 14.7.

From the above, it is seen that the presence of the chitosan glutamate crosslinking agent greatly improves the Absorbency Under Load value of low molecular weight carboxymethyl cellulose compared to nonheat-treated materials and heat-treated materials not containing a crosslinking agent.

The method according to the present invention is found to produce an improvement in AUL values in modified polysaccharides over a wide range of molecular weights. While high molecular weight modified polysaccharides are generally preferred, it is important that improvements in low molecular weight modified polysaccharides can be achieved. Low molecular weight modified polysaccharides are generally cheaper than high molecular weight modified polysaccharides. Accordingly, there is an economic advantage for employing low molecular weight modified polysaccharides. Further, it is possible to work with aqueous solutions containing relatively high concentrations of low molecular weight modified polysaccharides compared to aqueous solutions containing high concentrations of high molecular weight modified polysaccharides. This is because aqueous solutions of high molecular weight modified polysaccharides exhibit a high viscosity compared to an aqueous solution containing the same concentration of low molecular weight modified polysaccharides. Again, for reasons of efficiency, it is often desirable to form an aqueous solution comprising the highest concentration of modified polysaccharides possible while still being able to effectively work with the aqueous solution.

### Example No. 5

To determine the aging characteristics of absorbent material according to the present invention and comparative absorbent material, the following samples are provided:
Sample No. 107 is prepared exactly like Sample No. 2.
Sample No. 108 is prepared exactly like Sample Nos. 25-28, except the material is heated at 150°C. for 70 minutes.
Sample No. 109 is prepared exactly like Sample No. 42. A different initial AUL value is obtained.
Sample No. 110 is prepared exactly like Sample No. 24, except the material is heated for 20 minutes.
Sample No. 111 is prepared according to the method of Example 1 employing ethylene glycol as the crosslinking agent. The material is heat-treated at 170°C. for 30 minutes.
Sample Nos. 107-111 were placed in a temperature and humidity controlled environment. The temperature was maintained at 24°C. and the humidity was maintained at 30 percent relative humidity. The samples were tested for AUL value at various points throughout the 60-day aging study. The results are set forth in Table 4. The reported "AUL retention" is the 60-day AUL reported as a percentage of day 0 (starting) AUL. That is, 60-day AUL divided by 0 day AUL.

**TABLE 4**

| AUL Value (g/g) | | | | | | | | |
|---|---|---|---|---|---|---|---|---|
| Sample No. | 0 days | 4 days | 8 days | 12 days | 26 days | 50 days | 60 days | AUL Retention |
| 107* | 22.3 | 17.0 | 14.2 | 17.7 | 12.1 | 7.6 | 7.7 | 34.5 |
| 108 | 25.9 | 20.9 | 22.5 | 22.4 | 20.9 | 20.9 | 21.5 | 83.1 |
| 109 | 24.5 | 22.7 | 21.8 | 21.8 | 19.3 | 18.1 | 18.8 | 76.7 |
| 110 | 21.8 | 20.4 | 20.9 | 19.7 | 17.0 | 15.1 | 16.2 | 74.3 |
| 111 | 21.5 | 20.1 | 17.9 | 18.5 | 16.3 | 16.1 | 15.8 | 73.5 |

| | | | | | | | | |
|---|---|---|---|---|---|---|---|---|
| *Not an example of the present invention | | | | | | | | |

As can be seen from reference to Table 4, while heat-treatment alone can, in the absence of a crosslinking agent, provide an acceptable starting AUL, the 60-day AUL retention is only 34.5 percent (Sample No. 107). The presence of a crosslinking agent according to the present invention provides an improved 60-day AUL retention (Sample Nos. 108-111).

## Claims

1. A method for producing a water-swellable, water-insoluble modified polysaccharide, the method comprising the following steps:
forming a mixture comprising a water-soluble modified polysaccharide, water and a crosslinking agent wherein said mixture, comprising said water-soluble modified polysaccharide, water, and a crosslinking agent, is basic (pH > 7), wherein the water-soluble modified polysaccharide dissolves into the water and wherein the modified polysaccharide is selected from carboxylated, sulfonated, sulfated, and phosphated derivatives of polysaccharides, their salts, and mixtures thereof;
recovering said modified polysaccharide and said crosslinking agent from said mixture by evaporative drying; and
heat-treating said recovered modified polysaccharide and said crosslinking agent at a temperature between 100°C and 200°C for 20 to 100 minutes to crosslink said modified polysaccharide to render it water-swellable and water-insoluble, as well as to provide it with an Absorbency Under Load, as defined in the description and Fig. 1, of at least 17, the water-swellable, water-insoluble modified polysaccharide retaining greater than 50 percent of its Absorbency Under Load after aging for 60 days at 24°C and 30 percent relative humidity.

2. The method according to claim 1 wherein the modified polysaccharide is a carboxyalkyl polysaccharide.

3. The method according to claim 2 wherein the carboxyalkyl polysaccharide is a carboxyalkyl cellulose.

4. The method according to claim 3 wherein the carboxyalkyl cellulose is carboxymethyl cellulose.

5. The method according to any one of the preceding claims wherein the crosslinking agent is an organic compound comprising at least two functional groups capable of reacting with a carboxyl or hydroxyl group of a polysaccharide.

6. The method according to any one of the preceding claims wherein said crosslinking agent is selected from diamines, polyamines, diols, polyols and mixtures thereof.

7. The method according to claim 6 wherein the corsslinking agent is selected from chitosan glutamate, type A gelatin, diethylenetriamine, ethylene glycol, butylene glycol, polyvinyl alcohol, hyaluronic acid, polyethylene imine, and mixtures thereof.

8. The method according to any one of the preceding claims wherein said recovered modified polysaccharide is heat-treated under conditions sufficient to cause the modified polysaccharide to crosslink involving self-crosslinking which occurs through esterification.

9. The method according to any one of claims 1 to 6 wherein said crosslinking agent is a diamine or polyamine and the recovered modified polysaccharide is heat-treated to cause crosslinking formed by esterification and amidation.

10. The method according to claim 4 or any claim dependent on claim 4 wherein the carboxymethyl cellulose is heat-treated at a temperature and for a time sufficient to provide it with an Absorbency Under Load, as defined in the description and Fig. 1, of at least 20.

11. The method according to any one of the preceding claims wherein the heat-treated modified polysaccharide has an Absorbency Under Load, as defined in the description and Fig. 1, at least 10 percent greater than an identical modified polysaccharide without a crosslinking agent.

12. The method according to claim 11 wherein the heat-treated modified polysaccharide has an Absorbency Under Load, as defined in the description and Fig. 1, at least 20 percent greater than an identical modified polysaccharide without a crosslinking agent.

13. A method for producing a water-swellable, water-insoluble carboxyalkyl cellulose, especially according to claim 3 and any one of the claims depending on claim 3, the method comprising the following steps:
forming a mixture comprising a water-soluble carboxyalkyl cellulose, water, and a crosslinking agent selected from organic compounds comprising at least two functional groups capable of reacting with a carboxyl or hydroxyl group of a carboxyalkyl cellulose wherein said mixture, comprising said water-soluble modified polysaccharide, water, and a crosslinking agent, is basic (pH >7), wherein the water-soluble carboxyalkyl cellulose dissolves into the water;
recovering said carboxyalkyl cellulose and said crosslinking agent from said mixture by evaporative drying; and
heat-treating said recovered carboxyalkyl cellulose and said crosslinking agent at a temperature between 100°C and 200°C for 20 to 100 minutes to crosslink said carboxyalkyl cellulose to render it water-swellable and water-insoluble, as well as to provide it with an Absorbency Under Load, as defined in the description and Fig. 1, of at least 17, the water-swellable, water-insoluble carboxyalkyl cellulose retaining greater than 50 percent of its Absorbency Under Load after aging for 60 days at 24°C and 30 percent relative humidity.

14. A water-swellable, water-insoluble modified polysaccharide obtainable by the method of any one of the preceding claims.

15. The water-swellable, water-insoluble modified polysaccharide of claim 14 which retains greater than 70 percent of its Absorbency Under Load, as defined in the description and Fig. 1, after aging for 60 days at 24°C and 30 percent relative humidity.

16. A water-swellable, water-insoluble modified polysaccharide obtainable by a process according to claim 9 or any claim dependent on claim 9, **characterized in that** said modified polysaccharide possesses crosslinks formed by esterification and amidation.

17. The modified polysaccharide according to claim 16 wherein said modified polysaccharide is a carboxyalkyl polysaccharide.

18. The modified polysaccharide according to claim 17 wherein said carboxyalkyl polysaccharide is a carboxyalkyl cellulose.

19. The modified polysaccharide according to claim 18 wherein said carboxyalkyl cellulose is carboxymethyl cellulose.

20. The modified polysaccharide according to any one of claims 16 to 19 wherein said crosslinks formed by esterification result from crosslinks formed by self-crosslinking and the presence of a crosslinking agent other than a diamine or polyamine.

21. The modified polysaccharide according to any one of claims 16 to 20 further **characterized in that** the modified polysaccharide retains greater than 70 percent of its Absorbency Under Load, as defined in the description and Fig. 1, after aging for 60 days at 24°C and 30 percent relative humidity.

## Patentansprüche

1. Verfahren zur Herstellung eines wasserquellbaren, wasserunlöslichen modifizierten Polysaccharids, wobei das Verfahren folgende Schritte umfaßt:
Bildung eines Gemisches mit einem wasserlöslichen modifizierten Polysaccharid, Wasser und einem Vernetzungsmittel, wobei das Gemisch, das das wasserlösliche modifizierte Polysaccard, Wasser und ein Vernetzungsmittel umfasst, basisch (pH > 7) ist, wobei sich das wasserlösliche modifizierte Polysaccharid in dem Wasser auflöst, und wobei das modifizierte Polysaccharid aus carboxylierten, sulfonierten, sulfatierten und phosphatierten Derivaten von Polysacchariden, deren Salzen und Gemischen hiervon ausgewählt ist;
Rückgewinnung des modifizierten Polysaccharids und des Vernetzungsmittels aus dem Gemisch durch Verdunstungstrocknung; und
Wärmebehandlung des gewonnenen modifizierten Polysaccharids und des Vernetzungsmittels bei einer Temperatur zwischen 100°C und 200°C für 20 bis 100 Minuten, um das modifizierte Polysaccharid zu vernetzen, um es wasserquellbar und wasserunlöslich zu machen, und um ihm ein Absorptionsvermögen unter Belastung wie in der Beschreibung und in Fig. 1 definiert von mindestens 17 zu verleihen, wobei das wasserquellbare, wasserunlösliche modifizierte Polysaccharid mehr als 50 Prozent seines Absorptionsvermögens unter Belastung nach Altern über 60 Tage bei 24°C und 30 Prozent relativer Feuchtigkeit beibehält.

2. Verfahren gemäß Anspruch 1, bei dem das modifizierte Polysaccharid ein Carboxyalkylpolysaccharid ist.

3. Verfahren gemäß Anspruch 2, bei dem das Carboxyalkylpolysaccharid Carboxyalkylcellulose ist.

4. Verfahren gemäß Anspruch 3, bei dem die Carboxyalkylcellulose Carboxymethylcellulose ist.

5. Verfahren gemäß einem der vorhergehenden Ansprüche, bei dem das Vernetzungsmittel eine organische Verbindung mit mindestens zwei funktionellen Gruppen ist, welche zur Reaktion mit einer Carboxyl- oder Hydroxylgruppe eines Polysaccharids fähig sind.

6. Verfahren gemäß einem der vorhergehenden Ansprüche, bei dem das Vernetzungsmittel aus Diaminen, Polyaminen, Diolen, Polyolen und Gemischen hiervon ausgewählt ist.

7. Verfahren gemäß Anspruch 6, bei dem das Vernetzungsmittel aus Chitosanglutamat, Gelatine vom Typ A, Diethylentriamin, Ethylenglycol, Butylenglycol, Polyvinylalkohol, Hyaluronsäure, Polyethylenimin und Gemischen hiervon ausgewählt ist.

8. Verfahren gemäß einem der vorhergehenden Ansprüche, bei dem das gewonnene modifizierte Polysaccharid unter Bedingungen wärmebehandelt wird, welche ausreichen, eine Vernetzung des modifizierten Polysaccharids zu bewirken, welche eine Selbstvernetzung einschließt, welche durch Verestern erfolgt.

9. Verfahren gemäß einem der Ansprüche 1 bis 6, bei dem das Vernetzungsmittel ein Diamin oder Polyamin ist und das gewonnene modifizierte Polysaccharid wärmebehandelt wird, um eine Vernetzung, gebildet durch Veresterung und Amidierung, zu bewirken.

10. Verfahren gemäß Anspruch 4 oder einem von Anspruch 4 abhängigen Anspruch, bei dem die Carboxymethylcellulose bei einer Temperatur und über eine Zeitdauer wärmebehandelt wird, welche ausreichen, um ihr ein Absorptionsvermögen unter Belastung wie in der Beschreibung und in Fig. 1 definiert von mindestens 20 zu verleihen.

11. Verfahren gemäß einem der vorhergehenden Ansprüche, bei dem das wärmebehandelte modifizierte Polysaccharid ein Absorptionsvermögen unter Belastung wie in der Beschreibung und in Fig. 1 definiert von mindestens 10 Prozent mehr als ein identisches modifiziertes Polysaccharid ohne Vernetzungsmittel aufweist.

12. Verfahren gemäß Anspruch 11, bei dem das wärmebehandelte modifizierte Polysaccharid ein Absorptionsvermögen unter Belastung wie in der Beschreibung und in Fig. 1 definiert von mindestens 20 Prozent mehr als ein identisches modifiziertes Polysaccharid ohne Vernetzungsmittel aufweist.

13. Verfahren zur Herstellung einer wasserquellbaren, wasserunlöslichen Carboxyalkylcellulose, insbesondere gemäß Anspruch 3 und einem der von Anspruch 3 abhängigen Ansprüche, wobei das Verfahren folgende Schritte umfaßt:
Bildung eines Gemisches mit einer wasserlöslichen Carboxyalkylcellulose, Wasser und einem Vernetzungsmittel ausgewählt aus organischen Verbindungen mit mindestens zwei funktionellen Gruppen, welche zur Reaktion mit einer Carboxyl- oder Hydroxylgruppe einer Carboxyalkylcellulose fähig sind, wobei das Gemisch, das das wasserlösliche modifizierte Polysaccard, Wasser und ein Vernetzungsmittel umfasst, basisch (pH > 7) ist, wobei sich die wasserlösliche Carboxyalkylcellulose in dem Wasser auflöst;
Rückgewinnung der Carboxyalkylcellulose und des Vernetzungsmittels aus dem Gemisch durch Verdunstungstrocknung; und
Wärmebehandlung der gewonnenen Carboxyalkylcellulose und des Vernetzungsmittels bei einer Temperatur zwischen 100°C und 200°C für 20 bis 100 Minuten, um die Carboxyalkylcellulose zu vernetzen, um sie wasserquellbar und wasserunlöslich zu machen, und um ihr ein Absorptionsvermögen unter Belastung wie in der Beschreibung und in Fig. 1 definiert von mindestens 17 zu verleihen, wobei die wasserquellbare, wasserunlösliche Carboxyalkylcellulose mehr als 50 Prozent ihres Absorptionsvermögens unter Belastung nach Altern über 60 Tage bei 24°C und 30 Prozent relativer Feuchtigkeit beibehält.

14. Durch das Verfahren gemäß einem der vorhergehenden Ansprüche erhältliches wasserquellbares, wasserunlösliches modifiziertes Polysaccharid.

15. Wasserquellbares, wasserunlösliches modifiziertes Polysaccharid gemäß Anspruch 14, das mehr als 70 Prozent seines Absorptionsvermögens unter Belastung wie in der Beschreibung und in Fig. 1 definiert nach Altern über 60 Tage bei 24°C und 30 Prozent relativer Feuchtigkeit beibehält.

16. Wasserquellbares, wasserunlösliches modifiziertes Polysaccharid, erhältlich durch ein Verfahren gemäß Anspruch 9 oder einem von Anspruch 9 abhängigen Anspruch, **dadurch gekennzeichnet, daß** das modifizierte Polysaccharid Vernetzungsstellen, gebildet durch Veresterung und Amidierung, aufweist.

17. Modifiziertes Polysaccharid gemäß Anspruch 16, bei dem das modifizierte Polysaccharid ein Carboxyalkylpolysaccharid ist.

18. Modifiziertes Polysaccharid gemäß Anspruch 17, bei dem das Carboxyalkylpolysaccharid eine Carboxyalkylcellulose ist.

19. Modifiziertes Polysaccharid gemäß Anspruch 18, bei dem die Carboxyalkylcellulose Carboxymethylcellulose ist.

20. Modifiziertes Polysaccharid gemäß einem der Ansprüche 16 bis 19, bei dem die durch Verestern gebildeten Verbindungsstellen aus Verbindungsstellen, welche durch Selbstvernetzung gebildet sind, und dem Vorhandensein eines Vernetzungsmittels mit Ausnahme von Diamin oder Polyamin herrühren.

21. Modifiziertes Polysaccharid gemäß einem der Ansprüche 16 bis 20, des weiteren **dadurch gekennzeichnet, daß** das modifizierte Polysaccharid mehr als 70 Prozent seines Absorptionsvermögens unter Belastung wie in der Beschreibung und in Fig. 1 definiert nach Altern über 60 Tage bei 24°C und 30 Prozent relativer Feuchtigkeit beibehält.

## Revendications

1. Procédé de production d'un polysaccharide modifié, gonflable dans l'eau et insoluble dans l'eau, le procédé comprenant les étapes suivantes :
- la formation d'un mélange comprenant un polysaccharide modifié soluble dans l'eau, de l'eau et un agent de réticulation, dans lequel ledit mélange constitué par ledit polysaccharide modifié soluble dans l'eau, l'eau et un agent de réticulation, est basique (pH>7), le polysaccharide modifié soluble dans l'eau se dissout dans l'eau, et le polysaccharide modifié est choisi parmi les dérivés carboxylés, sulfonatés, sulfatés et phosphatés de polysaccharides, leurs sels et leurs mélanges ;
- la récupération dudit polysaccharide modifié et dudit agent de réticulation depuis ledit mélange par séchage par évaporation ; et
- le traitement thermique dudit polysaccharide modifié récupéré et dudit agent de réticulation, à une température comprise entre 100°C et 200°C pendant de 20 à 100 minutes pour réticuler ledit polysaccharide modifié, afin de le rendre gonflable dans l'eau et insoluble dans l'eau, et pour lui conférer une Capacité d'Absorption sous Charge, telle que définie dans la description et à la Figure 1, d'au moins 17, le polysaccharide modifié gonflable dans l'eau et insoluble dans l'eau conservant plus de 50% de sa Capacité d'Absorption sous Charge après vieillissement pendant 60 jours à 24°C et dans une humidité relative de 30%.

2. Procédé selon la revendication 1, dans lequel le polysaccharide modifié est un carboxyalkyl polysaccharide.

3. Procédé selon la revendication 2, dans lequel le carboxyalkyl polysaccharide est une carboxyalkyl cellulose.

4. Procédé selon la revendication 3, dans lequel la carboxyalkyl cellulose est la carboxyméthyl cellulose.

5. Procédé selon l'une quelconque des revendications précédentes, dans lequel l'agent de réticulation est un composé organique comprenant au moins deux groupes fonctionnels capables de réagir avec un groupe carboxyle ou hydroxyle d'un polysaccharide.

6. Procédé selon l'une quelconque des revendications précédentes, dans lequel ledit agent de réticulation est choisi parmi les diamines, les polyamines, les diols, les polyols et leurs mélanges.

7. Procédé selon la revendication 6, dans lequel l'agent de réticulation est choisi parmi le glutamate de chitosane, la gélatine de type A, la diéthylènetriamine, l'éthylène glycol, le butylène glycol, l'alcool polyvinylique, l'acide hyaluronique, la polyéthylène imine, et leurs mélanges.

8. Procédé selon l'une quelconque des revendications précédentes, dans lequel ledit polysaccharide modifié récupéré est traité thermiquement dans des conditions suffisantes pour réticuler le polysaccharide modifié, impliquant une auto-réticulation qui se produit par estérification.

9. Procédé selon l'une quelconque des revendications 1 à 6, dans lequel ledit agent de réticulation est une diamine ou une polyamine et le polysaccharide modifié récupéré est traité thermiquement pour provoquer une réticulation par estérification et amidation.

10. Procédé selon la revendication 4 ou une revendication quelconque dépendant de la revendication 4, dans lequel la carboxyméthyl cellulose est traitée thermiquement à une température, et pendant une période de temps, suffisantes pour lui conférer une Capacité d'Absorption sous Charge, telle que définie dans la description et à la Figure 1, d'au moins 20.

11. Procédé selon l'une quelconque des revendications précédentes, dans lequel le polysaccharide modifié traité thermiquement a une Capacité d'Absorption sous Charge, telle que définie dans la description et à la Figure 1, d'au moins 10% supérieure à celle d'un polysaccharide modifié identique, ne comprenant pas d'agent de réticulation.

12. Procédé selon la revendication 11, dans lequel le polysaccharide modifié traité thermiquement a une Capacité d'Absorption sous Charge, telle que définie dans la description et à la Figure 1, d'au moins 20% supérieure à celle d'un polysaccharide modifié identique, ne comprenant pas d'agent de réticulation.

13. Procédé de production d'une carboxyalkyl cellulose gonflable dans l'eau et insoluble dans l'eau, en particulier selon la revendication 3 et l'une quelconque des revendications dépendant de la revendication 3, le procédé comprenant les étapes suivantes :
- la formation d'un mélange comprenant une carboxyalkyl cellulose soluble dans l'eau, de l'eau et un agent de réticulation choisi parmi les composés organiques comprenant au moins deux groupes fonctionnels capables de réagir avec un groupe carboxyle ou hydroxyle d'une carboxyalkyl cellulose, ledit mélange constitué par ledit polysaccharide modifié soluble dans l'eau, l'eau et un agent de réticulation étant basique (pH>7), la carboxyalkyl cellulose soluble dans l'eau se dissolvant dans l'eau ;
- la récupération de ladite carboxyalkyl cellulose et dudit agent de réticulation depuis ledit mélange par séchage par évaporation ; et
- le traitement thermique de ladite carboxyalkyl cellulose récupérée et dudit agent de réticulation, à une température comprise entre 100°C et 200°C pendant de 20 à 100 minutes pour réticuler ladite carboxyalkyl cellulose afin de la rendre gonflable dans l'eau et insoluble dans l'eau et de lui conférer une Capacité d'Absorption sous Charge, telle que définie dans la description et à la Figure 1, d'au moins 17, la carboxyalkyl cellulose gonflable dans l'eau et insoluble dans l'eau conservant plus de 50% de sa Capacité d'Absorption sous Charge après vieillissement pendant 60 jours à 24°C et dans une humidité relative de 30%.

14. Polysaccharide modifié gonflable dans l'eau et insoluble dans l'eau, obtenu par le procédé selon l'une quelconque des revendications précédentes.

15. Polysaccharide modifié gonflable dans l'eau et insoluble dans l'eau selon la revendication 14, qui conserve plus de 70% de sa Capacité d'Absorption sous Charge, telle que définie dans la description et à la Figure 1, après vieillissement pendant 60 jours à 24°C et dans une humidité relative de 30%.

16. Polysaccharide modifié gonflable dans l'eau et insoluble dans l'eau, susceptible d'être obtenu par le procédé selon la revendication 9 ou selon toute revendication dépendant de la revendication 9, **caractérisé en ce que** ledit polysaccharide modifié possède des ponts formés par estérification et amidation.

17. Polysaccharide modifié selon la revendication 16, dans lequel ledit polysaccharide modifié est un carboxyalkyl polysaccharide.

18. Polysaccharide modifié selon la revendication 17, dans lequel ledit carboxyalkyl polysaccharide est une carboxyalkyl cellulose.

19. Polysaccharide modifié selon la revendication 18, dans lequel ladite carboxyalkyl cellulose est la carboxyméthyl cellulose.

20. Polysaccharide modifié selon l'une quelconque des revendications 16 à 19, dans lequel lesdits ponts formés par estérification résultent de ponts formés par auto-réticulation et de la présence d'un agent de réticulation autre qu'une diamine ou une polyamine.

21. Polysaccharide modifié selon l'une quelconque des revendications 16 à 20, **caractérisé en outre en ce que** le polysaccharide modifié conserve plus de 70% de sa Capacité d'Absorption sous Charge, telle que définie dans la description et à la Figure 1, après vieillissement pendant 60 jours à 24°C et dans une humidité relative de 30%.
